(19) Europäisches Patentamt
European Patent Office
Office européen des brevets

(11) **EP 3 757 561 B1**

(12) **EUROPEAN PATENT SPECIFICATION**

(45) Date of publication and mention
of the grant of the patent:
**29.04.2026 Bulletin 2026/18**

(21) Application number: **18906758.0**

(22) Date of filing: **21.12.2018**

(51) International Patent Classification (IPC):
***G01N 33/49*** *(2006.01)*     ***G01N 27/416*** *(2006.01)*

(52) Cooperative Patent Classification (CPC):
**G01N 27/4163; G01N 33/492**

(86) International application number:
**PCT/JP2018/047389**

(87) International publication number:
**WO 2019/163281 (29.08.2019 Gazette 2019/35)**

(54) **AUTOMATED ANALYZER AND AUTOMATIC ANALYSIS METHOD**

AUTOMATISIERTER ANALYSATOR UND AUTOMATISCHES ANALYSEVERFAHREN

ANALYSEUR AUTOMATISÉ ET PROCÉDÉ D'ANALYSE AUTOMATIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.02.2018 JP 2018030892**

(43) Date of publication of application:
**30.12.2020 Bulletin 2020/53**

(73) Proprietor: **Hitachi High-Tech Corporation
Minato-ku
Tokyo 105-6409 (JP)**

(72) Inventors:
• **TAKEUCHI, Miwa
Tokyo 105-8717 (JP)**
• **USHIKU, Emiko
Tokyo 105-8717 (JP)**
• **TAKAHASHI, Kenichi
Tokyo 105-8717 (JP)**

(74) Representative: **MERH-IP Matias Erny Reichl
Hoffmann
Patentanwälte PartG mbB
Paul-Heyse-Straße 29
80336 München (DE)**

(56) References cited:
**EP-A2- 0 667 522          WO-A1-2015/008517
WO-A1-2017/059037     JP-A- 2002 228 629
JP-A- 2002 228 629       JP-A- 2012 058 117
JP-A- 2017 146 307       JP-A- H07 167 818
JP-A- H07 167 818        JP-A- H07 253 409
JP-A- H07 325 063        JP-A- S61 195 343**

**Description**

Technical Field

The present invention relates to an automatic analysis apparatus.

Background Art

In a clinical examination, the target ion concentrations of sodium ions, potassium ions, chlorine ions, and the like contained in a sample such as serum or urine are measured using an ion selection electrode. In the sample, various ions other than the target ions derived from an organism are mixed. In addition, since commercially-available calibration liquids and quality control samples (QC samples) are artifacts, some of them contain components that do not exist in actual serum or have a concentration composition apart from a standard value in clinical practice.

When the ion concentration in a biological sample is measured using the ion selection electrode, the ion selection electrode reacts to ions (coexisting ions) other than the target ions as described above in some cases. At this time, the ion selection electrode outputs the combined concentration of the target ions and the coexisting ions as a detection result of the target ions. A degree at which the ion selection electrode reacts to the coexisting ions is represented as a selection coefficient.

Each ion selection electrode has an individual difference in the selection coefficient, and is changed depending on hours of use or the number of times of use. If the selection coefficient differs, a degree of responding to the coexisting ions also differs, and thus a measurement value fluctuates. Accordingly, a measurement error of the target ion concentration occurs. An ideal ion selection electrode is not affected by ions other than the target ions at all, namely, the selection coefficient is 0. However, an electrode film specific in the target ions has not been found yet, and it is difficult to produce an ideal electrode in the current technology. Here, there is a technique to measure the ion concentration of the target ions in the ion selection electrode (refer to Patent Literature 1). Patent Literature 2 discloses a method of a selection coefficient for an interfering ion when an ion selective electrode used for measuring an ion concentration in a solution which is disturbed by ions other than the ion to be measured.

Citation List

Patent Literature

Patent Literature 1: Japanese Unexamined Patent Application Publication No. Hei 7 (1995)-167818
Patent Literature 2: JP H07 253409 A

Summary of Invention

Technical Problem

In the technique described in Patent Literature 1, an electrode for detecting target ions and an electrode for detecting coexisting ions are provided, and the influence of the coexisting ions is corrected by mutually using a detection result of each electrode. However, if extra electrodes are installed to correct the influence of the coexisting ions, extra space for the electrodes is consumed, resulting in an increase in running cost and device cost.

The present invention has been made in view of the problem as described above, and an object thereof is to provide an automatic analysis apparatus that can reduce the influence by coexisting ions without additionally providing ion selection electrodes other than those for detecting target ions.

Solution to Problem

An automatic analysis apparatus according to the present invention calculates a target ion concentration contained in a sample using a result of calculating a selection coefficient of an ion selection electrode and a result of measuring a coexisting ion concentration contained in the sample. The present invention is defined by the appended claims. Third and fourth embodiments are not encompassed by the wording of the claims but are considered useful for understanding the embodiments of the invention.

Advantageous Effects of Invention

According to an automatic analysis apparatus of the present invention, it is possible to provide an automatic analysis apparatus that reduces the influence on target ions by coexisting ions without additionally providing ion selection electrodes other than the target ions.

Brief Description of Drawings

Fig. 1 is an outline configuration diagram of an automatic analysis apparatus 100 according to a first embodiment.
Fig. 2 is a functional block diagram of the automatic analysis apparatus 100.
Fig. 3 is a functional block diagram of a correction unit 124.
Fig. 4 is a flowchart for describing a procedure in which the automatic analysis apparatus 100 calculates a target ion concentration in a sample.
Fig. 5 is a flowchart for describing a procedure in which the automatic analysis apparatus 100 measures the sodium ion concentration of a measurement sample 15 in a second embodiment.
Fig. 6 is a flowchart for describing a procedure in which the automatic analysis apparatus 100 according to a third embodiment calculates the target ion concentration in the sample.
Fig. 7 is a screen example for showing an electrode information list.
Fig. 8 is a screen example for showing temporal changes of a coexisting ion concentration in the sample.
Fig. 9 is a screen example for showing temporal changes of a selection coefficient of an electrode.

Description of Embodiments

<First Embodiment>

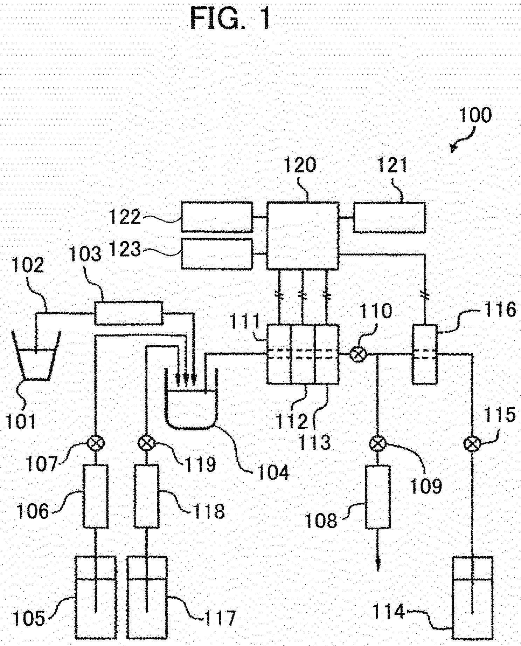

Fig. 1 is an outline configuration diagram of an automatic analysis apparatus 100 according to a first embodiment. The automatic analysis apparatus 100 is an apparatus that measures the concentration of target ions contained in a liquid sample. Hereinafter, a configuration of the automatic analysis apparatus 100 will be described.

A sample container 101 houses a biological sample (hereinafter, referred to as a sample) such as blood and urine. A sample dispensing nozzle 102 is immersed in the sample housed in the sample container 101. The sample dispensing nozzle 102 aspirates only a set amount of sample by an operation of a syringe 103 for sample dispensing nozzle, and discharges the same to a dilution tank 104. A diluted solution used for diluting the sample is housed in a diluted solution bottle 105. The diluted solution is fed to the dilution tank 104 by operations of a syringe for diluted solution 106 and a solenoid valve for diluted solution 107, and dilutes the sample in the dilution tank 104.

The sample diluted in the dilution tank 104 is aspirated to a sodium ion selection electrode 111, a potassium ion selection electrode 112, and a chlorine ion selection electrode 113 by operations of a sipper syringe 108, a solenoid valve for sipper syringe 109, and a pinch valve 110. A comparison electrode liquid housed in a comparison electrode liquid bottle 114 is aspirated to a comparison electrode 116 by operations of a solenoid valve for comparison electrode liquid 115, the sipper syringe 108, and the solenoid valve for sipper syringe 109. Electromotive force is measured between the comparison electrode 116 and each of the ion selection electrodes 111, 112, and 113.

In measurement of an internal standard solution used for obtaining a sample concentration, the internal standard solution housed in an internal standard solution bottle 117 is fed to the dilution tank 104 with the sample and the diluted solution removed by operations of a syringe for internal standard solution 118 and a solenoid valve for internal standard solution 119. The internal standard solution in the dilution tank 104 is aspirated to the sodium ion selection electrode 111, the potassium ion selection electrode 112, and the chlorine ion selection electrode 113 by operations of the sipper syringe 108, the solenoid valve for sipper syringe 109, and the pinch valve 110, and electromotive force between the selection electrodes and the comparison electrode 116 is measured. Electromotive force simply mentioned below means the electromotive force between the selection electrodes and the comparison electrode 116.

The sodium ion selection electrode 111, the potassium ion selection electrode 112, the chlorine ion selection electrode 113, and the comparison electrode 116 are connected to a control unit 120. The control unit 120 controls the entire operation of the automatic analysis apparatus 100, and not only measures the electromotive force generated among the electrodes, but also controls operations of each of the syringes 103, 106, 108, and 118 and each of the solenoid valves 107, 109, 110, 115, and 119. A storing unit 121, a display unit 122, and an input unit 123 are connected to the control unit 120. A user inputs information (sample type information and the like) of various parameters and the sample to be measured through the input unit 123 on the basis of a setting screen and the like displayed on the display unit 122. The storing unit 121 stores the input information. In addition to the information, the storing unit 121 stores various programs used for measuring the sample and measurement results.

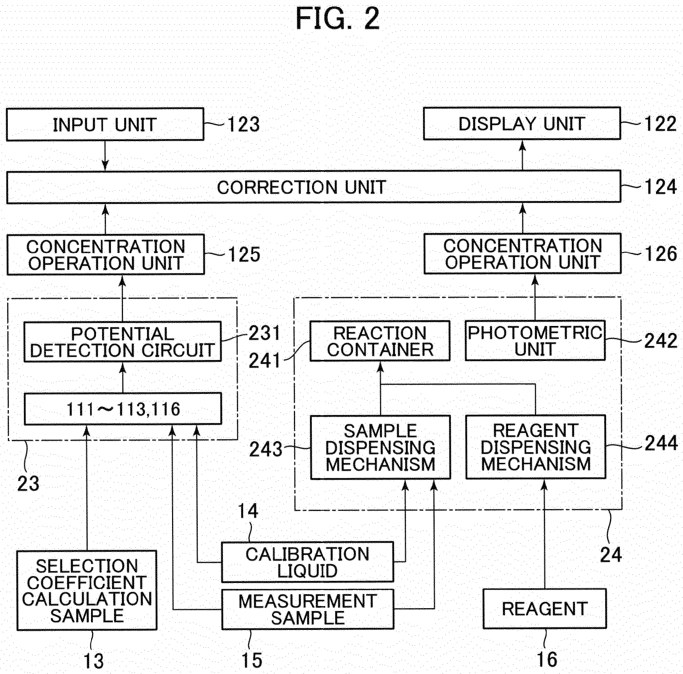

Fig. 2 is a functional block diagram of the automatic analysis apparatus 100. The automatic analysis apparatus 100 includes, as functional units for detecting the ion concentration in the sample, an electrolyte unit 23 and a measurement unit 24. The electrolyte unit 23 detects the ion concentration using the ion selection electrodes, and the measurement unit 24 measures the ion concentration using a color reaction.

The electrolyte unit 23 is configured using a potential detection circuit 231, the sodium ion selection electrode 111, the potassium ion selection electrode 112, the chlorine ion selection electrode 113, and the comparison electrode 116. The electrolyte unit 23 has a flow channel that allows the sample to pass through to be supplied to each electrode. The potential detection circuit 231 obtains a voltage output from each ion selection electrode as a detection result of the ion concentration by measuring a potential difference between each ion selection electrode and the comparison electrode 116. A concentration operation unit (calculation unit) 125 will be described later.

The measurement unit 24 includes a reaction container 241, a photometric unit 242, a sample dispensing mechanism 243, and a reagent dispensing mechanism 244. The sample dispensing mechanism 243 dispenses the sample to the reaction container 241, and the reagent dispensing mechanism 244 dispenses a reagent 16 to the reaction container 241. The measurement unit 24 conducts measurement of the sample in the reaction container 241 on the basis of the color reaction to measure the ion concentration in the sample. A concentration operation unit (calculation unit) 126 will be described later.

A selection coefficient calculation sample 13, a calibration liquid (first sample) 14, and a measurement sample (second sample) 15 are samples having roles that are different from each other. A correction unit 124 calculates a target ion concentration using measurement results for these samples. The use of each sample and a procedure for calculating the target ion concentration using each sample will be described later.

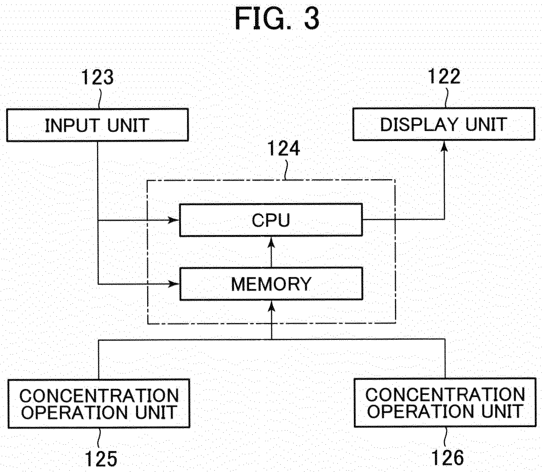

Fig. 3 is a functional block diagram of the correction unit 124. For convenience of description, the concentration operation units 125 and 126 are herein described as functional units different from the correction unit 124, but the concentration operation units 125 and 126 can be configured as functional units integrated with the correction unit 124. Hereinafter, a configuration of Fig. 3 will be described as a premise. The correction unit 124 can be configured using, for example, an operation device such as a CPU (Central Processing Unit) and a memory.

The concentration operation unit 125 calculates the ion concentration contained in the sample and the selection coefficients of the ion selection electrodes using a voltage output from the electrolyte unit 23, and stores the values into the memory of the correction unit 124. The concentration operation unit 126 calculates the ion concentration contained in the sample using the measurement result output from the measurement unit 24, and stores the value into the memory of the correction unit 124. The user inputs the input value of a target ion concentration to be described later into the automatic analysis apparatus 100 using the input unit 123. The display unit 122 displays the target ion concentration calculated by the correction unit 124 on the screen.

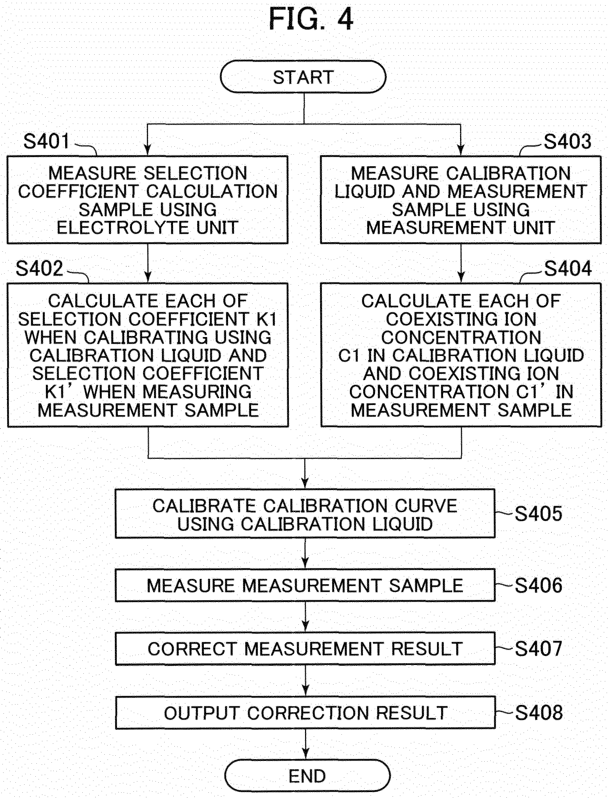

Fig. 4 is a flowchart for describing a procedure in which the automatic analysis apparatus 100 calculates the target ion concentration in the sample. Here, an example in which the detection characteristic line (hereinafter, the calibration curve) of sodium ions is calibrated using the calibration liquid 14 to calculate the sodium ion concentration contained in the measurement sample 15 will be described. In the following description, a coexisting ion detected by the sodium ion selection electrode 111 together with a sodium ion is assumed as $\alpha$.

(Fig. 4: Step S401)

The user detects the sodium ion concentration of the selection coefficient calculation sample 13 with the sodium ion concentration and the coexisting ion concentration already known using the sodium ion selection electrode 111 of the electrolyte unit 23. In the step, two samples having coexisting ion concentrations that are different from each other are prepared in advance as the selection coefficient calculation sample 13, and each sodium ion concentration is detected. Here, it is assumed that the sodium ion concentration is 140 mM (mol/L) and the $\alpha$ concentrations are 0 mM and 100 mM, respectively. In addition, it is assumed that the detection results of the sodium ion concentrations by the electrolyte unit 23 were 140 mM and 190 mM, respectively.

(Fig. 4: Step S401: Supplement)

In the step, it is necessary to detect the sodium ion concentrations using two kinds of selection coefficient calculation samples 13 for each of an electrode used when carrying out Step S405 and an electrode used when carrying out Step S406. Namely, four selection coefficient calculation samples 13 are basically needed. However, it is assumed that the same electrode is used in Step S405 and S406 to simplify the procedure in the flowchart. Thus, it is only necessary to carry out the step once using two kinds of selection coefficient calculation samples 13 for the same sodium ion selection electrode 111.

(Fig. 4: Step S402: Part 1)

The concentration operation unit 125 calculates the selection coefficient K1 of the sodium ion selection electrode 111 used when calibrating the calibration curve of the sodium ion concentration in Step S405. The concentration operation unit 125 stores the calculation result into the memory of the correction unit 124. The result of Step S401 shows K1 = (190-140)/(100-0) = 0.5. When calibrating the calibration curve in Step S405, it is significant in the step to preliminarily recognize the amount of coexisting ions taken in by the sodium ion selection electrode 111 as the detection result of the sodium ion concentration.

(Fig. 4: Step S402: Part 2)

The concentration operation unit 125 calculates the selection coefficient K1' of the sodium ion selection electrode 111 used when detecting the sodium ion concentration in the measurement sample 15 in Step S406. The concentration operation unit 125 stores the calculation result into the memory of the correction unit 124. Here, it is assumed that the same electrode is used in Step S405 and S406. Thus, K1 = K1' is satisfied. When measuring the measurement sample 15 in Step S406, it is significant in the step to preliminarily recognize the amount of coexisting ions taken in by the sodium ion selection electrode 111 as the detection result of the sodium ion concentration.

(Fig. 4: Steps S403 to S404)

In parallel with Step S401, the user measures a coexisting ion concentration contained in the calibration liquid 14 and a coexisting ion concentration contained in the measurement sample 15 using the measurement unit 24 (S403). The concentration operation unit 126 calculates a coexisting ion concentration C1 contained in the calibration liquid 14 and a coexisting ion concentration C'1 contained in the measurement sample 15 using the measurement results by the measurement unit 24 (S404). The concentration operation unit 126 stores the calculation results into the memory of the correction unit 124. Here, it is assumed that C1 = 30 mM and C'1 = 10 mM were satisfied.

(Fig. 4: Step S405)

The user supplies the calibration liquid 14 to the electrolyte unit 23 to calibrate the calibration curve of the sodium ion selection electrode 111. In general, the sensitivity of the ion selection electrode is gradually changed due to temporal changes and the like. Thus, even if a calibration liquid with the ion concentration already known is measured, a measurement result to be obtained is different from the already-known concentration in some cases. Accordingly, a correct detection result can be obtained by correcting the calibration curve of the ion selection electrode using the measurement result of the calibration liquid. This work is referred to as calibration of the calibration curve. The ion concentration of the calibration liquid 14 is presented from the shipping source of the calibration liquid 14. The user inputs the value into the automatic analysis apparatus 100 to correct the calibration curve. The value of the ion concentration presented by the shipping source of the sample as described above is referred to as an input value.

(Fig. 4: Step S405: Supplement)

Although the user specifies the input value of the sodium ion concentration of the calibration liquid 14 in the step, the actual detection result output from the sodium ion selection electrode 111 is the amount of coexisting ions that have been taken in. For example, in the case where the input value of the sodium ion concentration of the calibration liquid 14 is 140 mM, the sodium ion selection electrode 111 outputs $140 + (K1 \times C1) = 155$ mM as the detection result. Since the detection result is corrected by an input value of 140 mM in the step, the calibration curve is shifted to the small-value side by the difference. Thus, the detection result of the sodium ion selection electrode 111 thereafter is smaller than the actual sodium ion concentration by 15 mM.

(Fig. 4: Step S406)

The user supplies the measurement sample 15 to the electrolyte unit 23 to measure the sodium ion concentration contained in the measurement sample 15. At this time, the sodium ion selection electrode 111 outputs the detection result obtained by taking in the coexisting ions. For example, in the case where the concentration operation unit 125 outputs the sodium ion concentration as 150 mM, the actual sodium ion concentration in the measurement sample 15 is $150 - (K'1 \times C'1) = 145$ mM.

(Fig. 4: Step S406: Supplement)

Between the time when the calibration curve of the sodium ion selection electrode 111 is calibrated in Step S405 and the time when the sodium ion concentration is measured in Step S406, the sodium ion selection electrode 111 may be exchanged or the same sodium ion selection electrode may be used without being exchanged.

(Fig. 4: Step S407)

The correction unit 124 applies the calibration curve calibrated to the small-value side under the influence of the coexisting ions and the result of detecting the extra sodium ion concentration under the influence of the coexisting ions to the following Formula 1, so that the sodium ion concentration of the measurement sample 15 is corrected.

(Fig. 4: Step S407: Calculation formula)

In the following Formula 1, $C_i$ denotes the i-th coexisting ion concentration of the calibration liquid 14, $K_i$ denotes a selection coefficient for the i-th coexisting ion of the electrode used at the time of calibration, $C'_i$ denotes the i-th coexisting ion concentration of the measurement sample 15, $K'_i$ denotes a selection coefficient for the i-th coexisting ion of the electrode used when measuring the measurement sample 15, and $\Sigma$ denotes the sum of coexisting ions having an influence on the target ion. Since the number of kinds of coexisting ions $\alpha$ is only one in the above example, i is equal to 1. In the case where the influence of a plurality of coexisting ions is considered, Steps S401 to S404 are performed for each coexisting ion. In this case, the selection coefficients $K_2$, $K_3$, ·and so on of the sodium ion selection electrode 111 and the coexisting ion concentrations $C_2$, $C_3$, ·and so on are obtained for each coexisting ion to be substituted into the Formula 1.

"sodium ion concentration after calibration" = "measurement value by sodium ion selection electrode" + $\Sigma[I = 1 \rightarrow n](C_i \times K_i) - \Sigma[I = 1 \rightarrow n](C'_i \times K'_i)$

Formula 1

(Fig. 4: Step S407: Calculation example)

"sodium ion concentration after calibration" = $150 + (C_1 \times K_1) - (C'_1 \times K'_1) = 150 + 0.5 \times 30 - 0.5 \times 10 = 150 + 15 - 5 = 160$

(Fig. 4: Step S408)

The display unit 122 displays the result of Step S407 on the screen. In place of or together with the display unit 122, an appropriate output format such as "(a) outputting data describing the calculation result" or "(b) print-out through a printer" may be used. The same applies to the following embodiments.

<First embodiment: Conclusion>

In a conventional automatic analysis apparatus, an error of a measurement value caused by coexisting ions is treated as a measurement error. However, there is also a possibility that such an error of the measurement value caused by coexisting ions causes an error that cannot be overlooked in clinical practice. On the contrary, in the automatic analysis apparatus 100 according to the first embodiment, the selection coefficient of the ion selection electrode is calculated in advance before measuring the measurement sample 15, and the influence on the measurement value caused by coexisting ions is calibrated using the selection coefficient. Therefore, a measurement value closer to the actual value can be calculated.

In the case where the coexisting ion concentration of the calibration liquid 14 is different from that of the measurement sample 15, the measurement value of the measurement sample 15 is deviated from an assay value. When the measurement value of the measurement sample 15 is out of the quality control range, the reliability of specimen measurement data of the day cannot be obtained. On the contrary, in the automatic analysis apparatus 100 according to the first embodiment, the deviation from the assay value caused by coexisting ions can be calibrated by measuring the coexisting ions of each of the calibration liquid 14 and the measurement sample 15 by the measurement unit 24. Thus, the reliability of specimen measurement data can be secured.

<Second Embodiment>

When the shipping source of a sample (calibration liquid) ships the sample, the ion concentration contained in the sample is presented as a value input to an automatic analysis apparatus 100. A process of deciding the input value in the shipping source is called as value setting. When setting the value, the ion concentration is measured using an automatic analysis apparatus owned by the shipping source. Thus, a measurement value is influenced by coexisting ions in some cases. Accordingly, a procedure of reducing the influence of coexisting ions at the time of the value setting using the automatic analysis apparatus 100 in a second embodiment will be described. The configuration of the automatic analysis apparatus 100 is the same as the first embodiment, and thus a processing procedure performed by the automatic analysis apparatus 100 at the time of the value setting will be mainly described below.

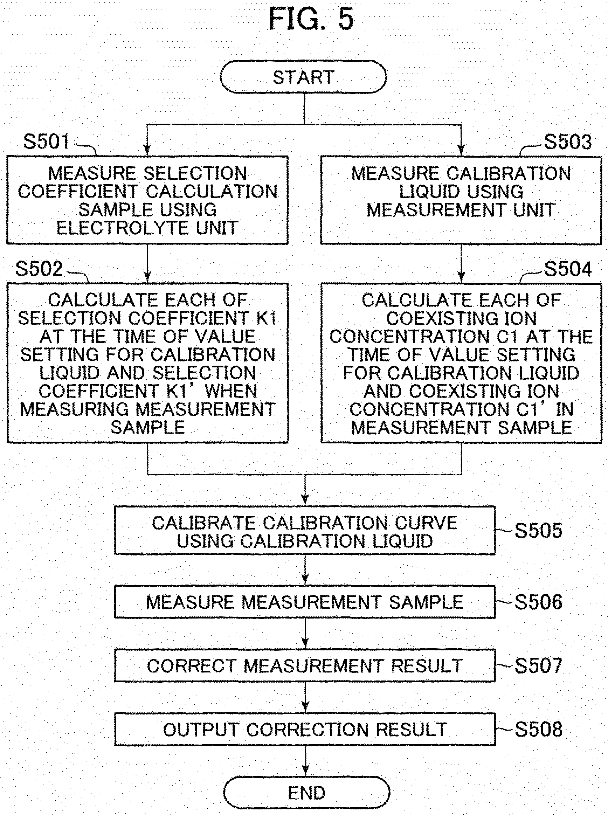

Fig. 5 is a flowchart for describing a procedure in which the automatic analysis apparatus 100 measures the sodium ion concentration of the measurement sample 15 in the second embodiment. The automatic analysis apparatus 100 reduces the influence of coexisting ions included in the input value specified by the shipping source using the procedure shown in Fig. 5. Hereinafter, each step of Fig. 5 will be described.

(Fig. 5: Step S501: Part 1)

As similar to Step S401, the user detects the sodium ion concentration of the selection coefficient calculation sample 13 with the sodium ion concentration and the coexisting ion concentration already known using the sodium ion selection electrode 111 used in Step S505. Here, it is assumed that the sodium ion concentration is 140 mM (mol/L) and the $\alpha$ concentrations are 0 mM and 100 mM, respectively. In addition, it is assumed that the detection results of the sodium ion concentrations by the electrolyte unit 23 were 140 mM and 170 mM, respectively.

(Fig. 5: Step S501: Part 2)

The shipping source of the calibration liquid 14 also measures the calibration liquid 14 as similar to the step at the stage before the shipment. Here, it is assumed that the sodium ion concentration is 140 mM (mol/L) and the $\alpha$ concentrations are 0 mM and 100 mM, respectively. In addition, it is assumed that the detection results of the sodium ion concentrations were 140 mM and 160 mM, respectively. It is only necessary to know each of the selection coefficient K'1 of the electrode used by the user and the selection coefficient K1 at the time of the value setting by the shipping source when performing the following steps. Thus, the electrode used by the user may be different from that used by the shipping source. Information sharing of each selection coefficient between the user and the shipping source will be described later.

(Fig. 5: Step S502: Part 1)

The concentration operation unit 125 calculates the selection coefficient K'1 of the sodium ion selection electrode 111 used when calibrating the calibration curve of the sodium ion concentration in Step S505. The concentration operation unit 125 stores the calculation result into the memory of the correction unit 124. The result of "Step S501: Part 1" shows K'1 = (170-140)/(100-0) = 0.3.

(Fig. 5: Step S502: Part 2)

The concentration operation unit 125 calculates the selection coefficient K1 when measuring the calibration liquid 14 for the value setting in the shipping source. The concentration operation unit 125 stores the calculation result into the memory of the correction unit 124. The result of "Step S501: Part 2" shows K1 = (160-140)/(100-0) = 0.2. For example, the result of "Step S501: Part 2" may be written on a document together with specifications and the like when the shipping source ships the calibration liquid 14, and the user may input the result into the automatic analysis apparatus 100. Alternatively, the shipping source may specify K1 itself, and may input the value into the automatic analysis apparatus 100.

(Fig. 5: Steps S503 to S504: Part 1)

In parallel with Step S501, the user measures the coexisting ion concentration contained in the calibration liquid 14 using the measurement unit 24 (S503). The concentration operation unit 126 calculates the coexisting ion concentration C'1 contained in the calibration liquid 14 using the measurement result by the measurement unit 24 (S504). The concentration operation unit 126 stores the calculation result into the memory of the correction unit 124. Here, it is assumed that C'1 = 30 mM was satisfied.

(Fig. 5: Steps S503 to S504: Part 2)

The shipping source of the calibration liquid 14 also measures the calibration liquid 14 as similar to S503 at the stage before the shipment. The concentration operation unit 126 receives the measurement result to calculate the coexisting ion concentration C1 in the shipping source. The measurement result in the shipping source is shared between the shipping source and the user as similar to S502, and it is only necessary for the user to input the result into the automatic analysis apparatus 100. Here, it is assumed that C1 = 30 mM was satisfied.

(Fig. 5: Step S505: Part 1)

As similar to Step S405, the user supplies the calibration liquid 14 to the electrolyte unit 23 to calibrate the calibration curve of the sodium ion selection electrode 111. For example, in the case where the input value of the sodium ion concentration of the calibration liquid 14 is 100 mM, it means that the measurement result obtained by taking in an extra amount of $(K1 \times C1) = 0.2 \times 30 = 6$ mM has been presented as an input value of 100 mM at the time of the value setting in the shipping source. Thus, the sodium ion concentration contained in the calibration liquid 14 is 100-6 = 94 mM.

(Fig. 5: Step S505: Part 2)

In the step, the sodium ion selection electrode 111 measures by taking in an extra amount of $(K'1 \times C'1) = 0.3 \times 30 = 9$ mM. Thus, 94 mM of the sodium ion concentration is measured as 103 mM.

(Fig. 5: Step S505: Part 3)

According to the above, since the sodium ion concentration measured as 103 mM is corrected by an input value of 100 mM, the calibration curve is shifted to the small-value side by the difference. Thus, the detection result of the sodium ion selection electrode 111 thereafter is smaller than the actual sodium ion concentration by 3 mM.

(Fig. 5: Step S506)

The user supplies the measurement sample 15 to the electrolyte unit 23 to measure the sodium ion concentration contained in the measurement sample 15. Here, it is assumed that the concentration operation unit 125 has calculated the sodium ion concentration as 122 mM.

(Fig. 5: Steps S507 to S508)

The correction unit 124 corrects the calibration curve calibrated to the small-value side under the influence of the coexisting ions in each of the shipping source and Step S505 in accordance with the following Formula 2 to calculate the correct sodium ion concentration of the measurement sample 15 (S507). The meaning of each coefficient of the Formula 2 is the same as the Formula 1. Step S508 is the same as Step S408.

(Fig. 5: Step S507: Calculation formula)

"correct sodium ion concentration" = "measurement value by sodium ion selection electrode" $-\Sigma[I = 1 \to n](C_i \times K_i) + \Sigma[I = 1 \to n] (C'_I \times K'_i)$ 

Formula 2

(Fig. 5: Step S507: Calculation formula)

"correct sodium ion concentration" = $122-(C1 \times K1) + (C'1 \times K'1) = 122-0.2 \times 30 + 0.3 \times 30 = 122-6 + 9 = 125$

<Second embodiment: Conclusion>

The automatic analysis apparatus 100 according to the second embodiment obtains the selection coefficient and the coexisting ion concentration of the calibration liquid 14 at the time of the value setting in the shipping source, and corrects the measurement result using the same. Thus, even if the selection coefficient and the coexisting ion concentration of the calibration liquid 14 in the shipping source are different from those when measuring the measurement sample 15 using the automatic analysis apparatus 100, the measurement error caused by these differences can be corrected.

<Third Embodiment>

In the above-described embodiments, the selection coefficient is calculated using the selection coefficient calculation sample 13, the coexisting ion concentration is measured using the measurement unit 24, and the measurement result is corrected using these values. If these values can be separately obtained, it is only necessary to input the obtained values into the automatic analysis apparatus 100 by omitting the measurement process. Accordingly, an operation procedure of the automatic analysis apparatus 100 in that case will be described in a third embodiment. The configuration of the automatic analysis apparatus 100 is the same as the first embodiment.

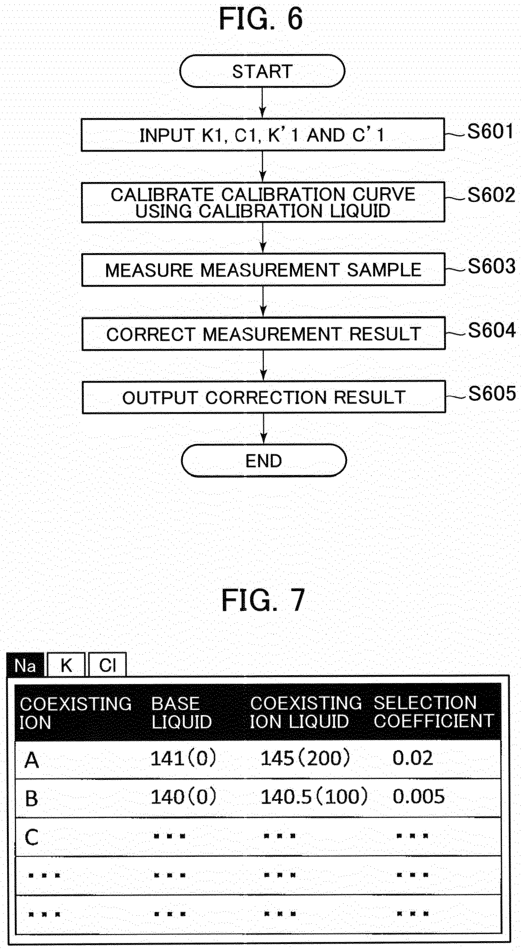

Fig. 6 is a flowchart for describing a procedure in which the automatic analysis apparatus 100 according to the third embodiment calculates the target ion concentration in the sample. Steps S602 to S605 are the same as Steps S405 to S408, and thus Step S601 will be described.

(Fig. 6: Step S601)

The user inputs the values of K1, C1, K'1, and C'1 that have been preliminarily obtained into the automatic analysis apparatus 100. The automatic analysis apparatus 100 performs Step S602 and those subsequent to Step S602 using these values. The user may manually input these values, or may supply these values using storage media or other data transmission means. Other appropriate methods may be used.

In the first embodiment, it is necessary to measure the selection coefficient and the coexisting ion concentrations of the calibration liquid 14 and the measurement sample 15 before measuring the measurement sample 15. On the contrary, the measurement work can be simplified in the third embodiment in the case where, for example, the above-described each value is provided from the manufacturer of the sample or in the case where it is conceivable that the selection coefficient and the coexisting ion concentrations are not changed from those previously measured. The procedure described in the second embodiment can be similarly replaced by Step S601 by omitting Steps S501 to S504 in the case where each value has been known in advance.

<Fourth Embodiment>

In a fourth embodiment, an example of a GUI (Graphical User Interface) provided by the display unit 122 will be described. The configuration of the automatic analysis apparatus 100 is the same as the first embodiment.

Fig. 7 is a screen example for showing an electrode information list. The screen has an electrode information list of: (a) a tab for selecting an electrode of target ions; and (b) coexisting ions/base liquid/coexisting ion liquid/selection coefficient. When the user selects the tab, the electrode of target ions is switched.

The coexisting ion column displays the name of coexisting ions for the electrode of the selected tab. The base liquid column displays the target ion concentration in the base liquid operated by the concentration operation unit 125. The value displayed in parenthesis is the coexisting ion concentration operated by the concentration operation unit 126. The coexisting ion liquid column displays the target ion concentration in the coexisting ion liquid operated by the concentration operation unit 125. The value displayed in parenthesis is the coexisting ion concentration operated by the concentration operation unit 126. The base liquid and the coexisting ion liquid correspond to two kinds of selection coefficient calculation samples 13 in Step S401. The selection coefficient column displays the selection coefficient calculated on the basis of information of the coexisting ion column and the base liquid column. The values displayed on the screen may be those obtained by other concentration measurement apparatuses, or those of concentrations that are provided from the manufacturer and input from the input unit 123.

The user can confirm the electrode information list most recently measured or input on the screen of Fig. 7. For example, if the selection coefficient is checked, the deterioration state of the electrode can be confirmed. This function allows to find error factors of the measurement value sooner.

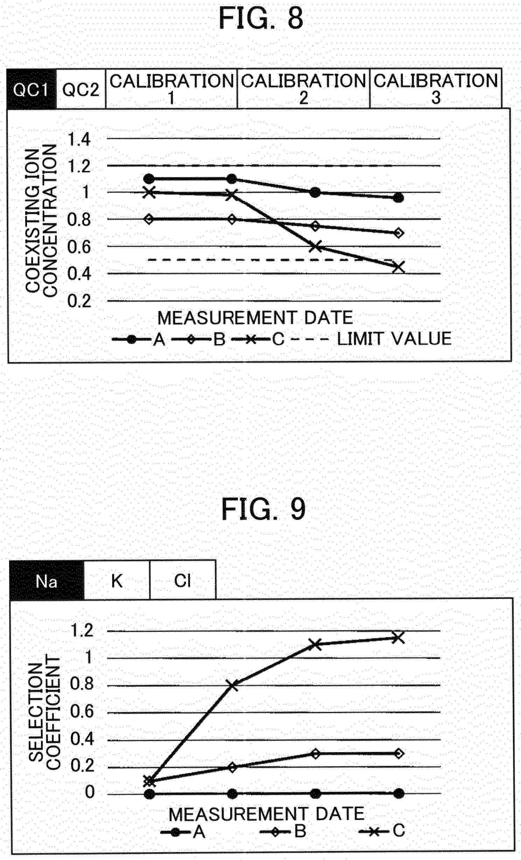

Fig. 8 is a screen example for showing temporal changes of the coexisting ion concentration in the sample. When the user selects a tab, the sample is switched, and the coexisting ion concentrations in the sample operated by the concentration operation unit 126 are displayed in time series. The vertical axis represents a coexisting ion concentration operated by the concentration operation unit 126 or a value input from the input unit 123. The horizontal axis represents time such as days or months or the number of measurement. If an allowable range for the coexisting ion concentration is set in advance and the measurement value is out of the allowable range, the CPU displays an alarm prompting to exchange the sample on, for example, the display unit 122. The alarm may be issued by other appropriate methods (for example, alarm sound or the like). The same applies to the other alarms.

The user monitors temporal changes of the coexisting ion concentration in the sample on the screen of Fig. 8.

Accordingly, a sample with the composition modified can be removed before the measurement. In addition, when the measurement value is out of the allowable range, an alarm is output. Accordingly, it is possible to prompt the user to remove the sample.

Fig. 9 is a screen example for showing temporal changes of the selection coefficient of the electrode. When the user selects a tab, the selection coefficients for coexisting ions of each electrode operated by the concentration operation unit 125 are displayed in time series. The vertical axis represents a selection coefficient operated by the concentration operation unit 125 or a value input from the input unit 123. The horizontal axis represents time such as days or months or the number of measurement. The graph is updated every time the electrolyte unit 23 measures. If a threshold value for the selection coefficient is set in advance and the selection coefficient exceeds the threshold value, the CPU displays an alarm prompting to exchange the electrode on, for example, the display unit 122.

The user monitors temporal changes of the selection coefficient for coexisting ions of each electrode on the screen of Fig. 9. Accordingly, an electrode having no lifetime can be removed before the measurement. In addition, when the measurement value is out of the allowable range, an alarm is output. Accordingly, it is possible to prompt the user to remove the electrode.

Each of the above-described configurations, functions, processing units, processing means, and the like may be realized using hardware by designing some or all thereof with, for example, integrated circuits. In addition, each of the above-described configurations, functions, and the like may be realized using software in such a manner that a processor interprets and executes a program realizing each function. Information of a program, a table, a file, and the like realizing each function can be stored in a storage device such as a memory, a hard disk, or an SSD (Solid State Drive), or a recording medium such as an IC card, or an SD card. In addition, the control lines and the information lines considered to be necessary in the explanation are shown, but all the control lines and the information lines in a product are not necessarily shown. In practice, almost all the configurations may be considered to be connected to each other.

List of Reference Signs

13: selection coefficient calculation sample
14: calibration liquid
15: measurement sample
23: electrolyte unit
24: measurement unit
100: automatic analysis apparatus
111: sodium ion selection electrode
112: potassium ion selection electrode
113: chlorine ion selection electrode
116: comparison electrode
122: display unit
123: input unit
124: correction unit
125: concentration operation unit
126: concentration operation unit

**Claims**

**1.** 1. An automatic analysis apparatus that is configured to measure the concentration of target ions contained in a sample, the apparatus comprising:

a measurement unit (24);
a first ion selection electrode (111; 112; 113) that is configured to detect the concentration of the target ions contained in the sample (14; 15); and
a calculation unit (124, 125, 126) that is configured to calculate the concentration of the target ions contained in the sample (14; 15),
wherein the calculation unit (124, 125, 126) is configured to obtain a detection result by the first ion selection electrode (111; 112; 113) and a measurement result by the measurement unit (24) that is configured to measure the concentration, $C_1$, $C'_1$, of coexisting ions contained in the sample (14; 15),
wherein the measurement unit (24) is configured to measure the concentration, $C_1$, $C'_1$, of the coexisting ions contained in the sample (14; 15) using a color reaction,
wherein the calculation unit (124, 125, 126) is configured to calculate a ratio of the coexisting ions detected by the first ion selection electrode (111; 112; 113) among those contained in the sample (14; 15) as a selection coefficient, $K1$, $K'1$, for the coexisting ions of the first ion selection electrode (111; 112; 113), and
wherein the calculation unit (124, 125, 126) is configured to calculate the concentration of the target ions contained in the sample (14; 15) using the detection result by the first ion selection electrode (111; 112; 113), the selection coefficient, $K1$, $K'1$, and the concentration, $C1$, $C'1$, of the coexisting ions measured by the measurement unit (24).

2.  The automatic analysis apparatus according to claim 1,

wherein the first ion selection electrode (111; 112; 113) and the measurement unit (24) are configured to measure each of a first sample (14) and a second sample (15) as the sample (14; 15),
wherein the calculation unit (124, 125, 126) is configured to calculate the selection coefficient for the coexisting ions contained in the first sample (14) as a first sample selection coefficient, $K1$, and is configured to calculate the selection coefficient for the coexisting ions contained in the second sample (15) as a second sample selection coefficient, $K'1$,
wherein the measurement unit (24) is configured to measure the concentration of the coexisting ions contained in the first sample (14) as a first sample coexisting ion concentration, $C1$, and is configured to measure the concentration of the coexisting ions contained in the second sample (15) as a second sample coexisting ion concentration, $C'1$,
wherein the calculation unit (124, 125, 126) is configured to calculate the concentration of the target ions contained in the second sample (15) using the first sample selection coefficient, $K1$, the second sample selection coefficient, $K'1$, the first sample coexisting ion concentration, $C1$, and the second sample coexisting ion concentration, $C'1$, and
wherein the calculation unit (124, 125, 126) is configured to calculate the concentration of the target ions contained in the second sample (15) by adding a value obtained by multiplying the first sample selection coefficient, $K1$, by the first sample coexisting ion concentration, $C1$, to a result of the concentration of the target ions contained in the second sample (15) measured by the first ion selection electrode (111; 112; 113) and by further subtracting a value obtained by multiplying the second sample selection coefficient, $K'1$, by the second sample coexisting ion concentration, $C'1$, from the result.

3.  The automatic analysis apparatus according to claim 2,

wherein the calculation unit (124, 125, 126) is configured to calibrate the first ion selection electrode (111; 112; 113) using a result of the concentration of the target ions contained in the first sample (14) detected by the first ion selection electrode (111; 112; 113) and a value preliminarily specified as the concentration of the target ions contained in the first sample (14),
wherein the calculation unit (124, 125, 126) is configured to obtain a result of the concentration of the target ions contained in the second sample (15) detected by the first ion selection electrode (111; 112; 113), and
wherein the calculation unit (124, 125, 126) is configured to calculate the concentration of the target ions contained in the second sample (15) by correcting a result of the calibration using the first sample selection coefficient, $K1$, the second sample selection coefficient, $K'1$, the first sample coexisting ion concentration, $C1$, and the second sample coexisting ion concentration, $C'1$.

4.  The automatic analysis apparatus according to claim 3,

wherein the first ion selection electrode (111; 112; 113) is configured to be exchanged between the time when the calculation unit calibrates the first ion selection electrode (111; 112; 113) and the time when the calculation unit obtains a result of detecting the concentration, $C'1$, of the target ions contained in the second sample (15), or wherein the same first ion selection electrode (111; 112; 113) is configured to be used at each of the time when the calculation unit calibrates the first ion selection electrode (111; 112; 113) and the time when the calculation unit obtains a result of detecting the concentration, $C'1$, of the target ions contained in the second sample (15).

5.  The automatic analysis apparatus according to claim 1,

wherein the sample (14; 15) comprises a first sample (14) and a second sample (15),
wherein in a manufacturing process before the first sample (14) is shipped, the calculation unit (124, 125, 126) is

**EP 3 757 561 B1**

configured to obtain a ratio of the coexisting ions detected by a second ion selection electrode that detects the concentration of the target ions among those contained in the first sample (14) as a first selection coefficient, K1, for the coexisting ions of the second ion selection electrode,
wherein each of the first ion selection electrode (111; 112; 113) and the measurement unit (24) are configured to measure the second sample (15),
wherein the calculation unit (124, 125, 126) is configured to calculate a ratio of the concentration of the target ions detected by the first ion selection electrode (111; 112; 113) among the coexisting ions contained in the second sample (15) as a second selection coefficient, K'1,
wherein the calculation unit (124, 125, 126) is configured to obtain a result of measuring the concentration of the coexisting ions contained in the first sample (14) in the manufacturing process as a first coexisting ion concentration, C1,
wherein the calculation unit (124, 125, 126) is configured to obtain a result of the concentration of the coexisting ions contained in the second sample (15) measured by the measurement unit (24) as a second coexisting ion concentration, C'1, and
wherein the calculation unit (124, 125, 126) is configured to calculate the concentration of the target ions contained in the second sample (15) using the first selection coefficient, K1, the second selection coefficient, K'1, the first coexisting ion concentration, C1, and the second coexisting ion concentration, C'1.

6. The automatic analysis apparatus according to claim 5,

wherein the calculation unit (124, 125, 126) is configured to calibrate the first ion selection electrode (111; 112; 113) using a result of the concentration of the target ions contained in the first sample (14) detected by the first ion selection electrode (111; 112; 113) and a value preliminarily specified as the concentration of the target ions contained in the first sample (14),
wherein the calculation unit (124, 125, 126) obtains a result of the concentration of the target ions contained in the second sample (15) detected by the first ion selection electrode (111; 112; 113), and
wherein the calculation unit (124, 125, 126) is configured to calculate the concentration of the target ions contained in the second sample (15) by correcting a result of the calibration using the first selection coefficient, K1, the second selection coefficient, K'1, the first coexisting ion concentration, C1, and the second coexisting ion concentration, C'1.

7. The automatic analysis apparatus according to claim 6,
wherein the calculation unit (124, 125, 126) is configured to calculate the concentration of the target ions contained in the second sample (15) by subtracting a value obtained by multiplying the first selection coefficient, K1, by the first coexisting ion concentration, C1, from a result of the concentration of the target ions contained in the second sample (15) measured by the first ion selection electrode (111; 112; 113) and by further adding a value obtained by multiplying the second selection coefficient, K'1, by the second coexisting ion concentration, C'1, to the result.

8. The automatic analysis apparatus according to claim 1, comprising an interface that is configured to input a detection result by the first ion selection electrode (111; 112; 113) and a measurement result by the measurement unit (24), wherein the calculation unit (124, 125, 126) is configured to calculate the concentration of the target ions contained in the sample (14; 15) using the detection result by the first ion selection electrode (111; 112; 113) input through the interface and the measurement result by the measurement unit (24) input through the interface.

9. The automatic analysis apparatus according to claim 1 or 5, comprising:

a storing unit (121) that is configured to store temporal changes of the concentration of the coexisting ions contained in the sample (14; 15); and
an output unit that is configured to output temporal changes of the concentration, C1, C'1, of the coexisting ions contained in the sample (14; 15).

10. The automatic analysis apparatus according to claim 1 or 5, comprising:

a storing unit (121) that is configured to store temporal changes of the selection coefficient of the first ion selection electrode (111; 112; 113); and
an output unit that is configured to output temporal changes of the selection coefficient, K1, K'1, of the first ion selection electrode (111; 112; 113).

12

11. The automatic analysis apparatus according to claim 1 or 5, comprising an alarm unit that is configured to output, when the concentration, C1, C' 1, of the coexisting ions contained in the sample (14; 15) or the selection coefficient, K1, K'1, of the first ion selection electrode (111; 112; 113) is out of a preliminarily set allowable range, an alarm of notifying the fact.

12. An automatic analysis method for measuring the concentration of target ions contained in a sample, the method comprising:

a step (S405, S406) of detecting the concentration of the target ions contained in the sample (14; 15) using a first ion selection electrode (111; 112; 113); and
a calculation step (S407) of calculating the concentration of the target ions contained in the sample (14; 15),
wherein in the calculation step (S407), a detection result by the first ion selection electrode (111; 112; 113) and a measurement result by a measurement unit (24) that measures the concentration, C1, C' 1, of coexisting ions contained in the sample (14; 15) are obtained,
wherein the measurement unit (24) measures the concentration, C1, C'1, of the coexisting ions contained in the sample (14; 15) using a color reaction,
wherein in the calculation step (S402), a ratio of the coexisting ions detected by the first ion selection electrode (111; 112; 113) among those contained in the sample (14; 15) is calculated as a selection coefficient, K1, K' 1, for the coexisting ions of the first ion selection electrode (111; 112; 113), and
wherein in the calculation step (S407), the concentration of the target ions contained in the sample (14; 15) is calculated using the detection result by the first ion selection electrode (111; 112; 113), the selection coefficient, K1, K'1, and the concentration, C1, C'1, of the coexisting ions measured by the measurement unit (24).

13. The automatic analysis method for measuring the concentration of target ions contained in a sample according to claim 12,

wherein the first ion selection electrode (111; 112; 113) and the measurement unit (24) measure each of a first sample (14) and a second sample (15) as the sample (14; 15),
wherein in the calculation step (S402), the selection coefficient for the coexisting ions contained in the first sample (14) is calculated as a first sample selection coefficient, K1, and the selection coefficient for the coexisting ions contained in the second sample (15) is calculated as a second sample selection coefficient, K'1,
wherein the measurement unit (24) measures the concentration of the coexisting ions contained in the first sample (14) as a first sample coexisting ion concentration, C1, and measures the concentration of the coexisting ions contained in the second sample (15) as a second sample coexisting ion concentration, C'1,
wherein in the calculation step (S407), the concentration of the target ions contained in the second sample (15) is calculated using the first sample selection coefficient, K1, the second sample selection coefficient, K'1, the first sample coexisting ion concentration, C1, and the second sample coexisting ion concentration, C'1, and
wherein the calculation unit (124, 125, 126) calculates the concentration of the target ions contained in the second sample (15) by adding a value obtained by multiplying the first sample selection coefficient, K1, by the first sample coexisting ion concentration, C1, to a result of the concentration of the target ions contained in the second sample (15) measured by the first ion selection electrode (111; 112; 113) and by further subtracting a value obtained by multiplying the second sample selection coefficient, K'1, by the second sample coexisting ion concentration, C'1, from the result.

14. The automatic analysis method according to claim 12,

wherein the sample (14; 15) comprises a first sample (14) and a second sample (15),
wherein in a manufacturing process before the first sample (14) is shipped, a ratio of the coexisting ions detected by a second ion selection electrode that detects the concentration of the target ions among those contained in the first sample (14) is obtained as a first selection coefficient, K1, for the coexisting ions of the second ion selection electrode in the calculation step,
wherein each of the first ion selection electrode (111; 112; 113) and the measurement unit (24) measures the second sample (15),
wherein in the calculation step (S502), a ratio of the concentration of the target ions detected by the first ion selection electrode (111; 112; 113) among the coexisting ions contained in the second sample (15) is calculated as a second selection coefficient, K'1,
wherein in the calculation step (S507), a result of measuring the concentration of the coexisting ions contained in the first sample (14) in the manufacturing process is obtained as a first coexisting ion concentration, C1,

wherein in the calculation step (S507), a result of the concentration of the coexisting ions contained in the second sample (15) measured by the measurement unit (24) is obtained as a second coexisting ion concentration, C' 1, and

wherein in the calculation step (S507), the concentration of the target ions contained in the second sample (15) is calculated using the first selection coefficient, K1, the second selection coefficient, K'1, the first coexisting ion concentration, C1, and the second coexisting ion concentration, C'1.

**Patentansprüche**

1.  1. Automatische Analysevorrichtung, die konfiguriert ist, um die Konzentration von in einer Probe enthaltenen Zielionen zu messen, wobei die Vorrichtung Folgendes umfasst:

    eine Messeinheit (24);
    eine erste Ionenauswahlelektrode (111; 112; 113), die konfiguriert ist, um die Konzentration der in der Probe (14; 15) enthaltenen Zielionen zu detektieren; und
    eine Berechnungseinheit (124, 125, 126), die konfiguriert ist, um die Konzentration der in der Probe (14; 15) enthaltenen Zielionen zu berechnen,
    wobei die Berechnungseinheit (124, 125, 126) konfiguriert ist, um ein Detektionsergebnis durch die erste Ionenauswahlelektrode (111; 112; 113) und ein Messergebnis durch die Messeinheit (24) zu erhalten, die konfiguriert ist, um die Konzentration, C1, C'1, von in der Probe (14; 15) enthaltenen koexistierenden Ionen zu messen,
    wobei die Messeinheit (24) konfiguriert ist, um die Konzentration, C1, C'1, der in der Probe (14; 15) enthaltenen koexistierenden Ionen unter Verwendung einer Farbreaktion zu messen,
    wobei die Berechnungseinheit (124, 125, 126) konfiguriert ist, um ein Verhältnis der durch die erste Ionenauswahlelektrode (111; 112; 113) detektierten koexistierenden Ionen unter denjenigen, die in der Probe (14; 15) enthalten sind, als einen Auswahlkoeffizienten, K1, K'1, für die koexistierenden Ionen der ersten Ionenauswahlelektrode (111; 112; 113) zu berechnen, und
    wobei die Berechnungseinheit (124, 125, 126) konfiguriert ist, um die Konzentration der in der Probe (14; 15) enthaltenen Zielionen unter Verwendung des Detektionsergebnisses durch die erste Ionenauswahlelektrode (111; 112; 113), des Auswahlkoeffizienten, K1, K'1, und der Konzentration, C1, C'1, der durch die Messeinheit (24) gemessenen koexistierenden Ionen zu berechnen.

2.  Automatische Analysevorrichtung nach Anspruch 1,

    wobei die erste Ionenauswahlelektrode (111; 112; 113) und die Messeinheit (24) konfiguriert sind, um jeweils eine erste Probe (14) und eine zweite Probe (15) als die Probe (14; 15) zu messen,
    wobei die Berechnungseinheit (124, 125, 126) konfiguriert ist, um den Auswahlkoeffizienten für die in der ersten Probe (14) enthaltenen koexistierenden Ionen als einen ersten Probenauswahlkoeffizienten, K1, zu berechnen, und konfiguriert ist, um den Auswahlkoeffizienten für die in der zweiten Probe (15) enthaltenen koexistierenden Ionen als einen zweiten Probenauswahlkoeffizienten, K'1, zu berechnen,
    wobei die Messeinheit (24) konfiguriert ist, um die Konzentration der in der ersten Probe (14) enthaltenen koexistierenden Ionen als eine erste koexistierende Probenionenkonzentration, C1, zu messen, und konfiguriert ist, um die Konzentration der in der zweiten Probe (15) enthaltenen koexistierenden Ionen als eine zweite koexistierende Probenionenkonzentration, C'1, zu messen,
    wobei die Berechnungseinheit (124, 125, 126) konfiguriert ist, um die Konzentration der in der zweiten Probe (15) enthaltenen Zielionen unter Verwendung des ersten Probenauswahlkoeffizienten, K1, des zweiten Probenauswahlkoeffizienten, K'1, der ersten koexistierenden Probenionenkonzentration, C1, und der zweiten koexistierenden Probenionenkonzentration, C'1, zu berechnen, und
    wobei die Berechnungseinheit (124, 125, 126) konfiguriert ist, um die Konzentration der in der zweiten Probe (15) enthaltenen Zielionen durch Addieren eines Werts, der durch Multiplizieren des ersten Probenauswahlkoeffizienten, K1, mit der ersten koexistierenden Probenionenkonzentration, C1, erhalten wird, zu einem Ergebnis der Konzentration der in der zweiten Probe (15) enthaltenen Zielionen, die durch die erste Ionenauswahlelektrode (111; 112; 113) gemessen wird, und durch weiteres Subtrahieren eines Werts, der durch Multiplizieren des zweiten Probenauswahlkoeffizienten, K'1, mit der zweiten koexistierenden Probenionenkonzentration, C'1, erhalten wird, von dem Ergebnis zu berechnen.

3.  Automatische Analysevorrichtung nach Anspruch 2,

wobei die Berechnungseinheit (124, 125, 126) konfiguriert ist, um die erste Ionenauswahlelektrode (111; 112; 113) unter Verwendung eines Ergebnisses der Konzentration der in der ersten Probe (14) enthaltenen Zielionen, die durch die erste Ionenauswahlelektrode (111; 112; 113) detektiert wird, und eines Werts, der vorläufig als die Konzentration der in der ersten Probe (14) enthaltenen Zielionen spezifiziert wird, zu kalibrieren,

wobei die Berechnungseinheit (124, 125, 126) konfiguriert ist, um ein Ergebnis der Konzentration der in der zweiten Probe (15) enthaltenen Zielionen, die durch die erste Ionenauswahlelektrode (111; 112; 113) detektiert wird, zu erhalten, und

wobei die Berechnungseinheit (124, 125, 126) konfiguriert ist, um die Konzentration der in der zweiten Probe (15) enthaltenen Zielionen durch Korrigieren eines Ergebnisses der Kalibrierung unter Verwendung des ersten Probenauswahlkoeffizienten, K1, des zweiten Probenauswahlkoeffizienten, K'1, der ersten koexistierenden Probenionenkonzentration, C1, und der zweiten koexistierenden Probenionenkonzentration, C'1, zu berechnen.

**4.** Automatische Analysevorrichtung nach Anspruch 3,

wobei die erste Ionenauswahlelektrode (111; 112; 113) konfiguriert ist, um zwischen der Zeit, zu der die Berechnungseinheit die erste Ionenauswahlelektrode (111; 112; 113) kalibriert, und der Zeit, zu der die Berechnungseinheit ein Ergebnis des Detektierens der Konzentration, C'1, der in der zweiten Probe (15) enthaltenen Zielionen erhält, ausgetauscht zu werden, oder

wobei dieselbe erste Ionenauswahlelektrode (111; 112; 113) konfiguriert ist, um zu jeder der Zeit, zu der die Berechnungseinheit die erste Ionenauswahlelektrode (111; 112; 113) kalibriert, und der Zeit, zu der die Berechnungseinheit ein Ergebnis des Detektierens der Konzentration, C'1, der in der zweiten Probe (15) enthaltenen Zielionen erhält, verwendet zu werden.

**5.** Automatische Analysevorrichtung nach Anspruch 1,

wobei die Probe (14; 15) eine erste Probe (14) und eine zweite Probe (15) umfasst,

wobei in einem Herstellungsprozess, bevor die erste Probe (14) versandt wird, die Berechnungseinheit (124, 125, 126) konfiguriert ist, um ein Verhältnis der durch eine zweite Ionenauswahlelektrode, die die Konzentration der Zielionen unter denjenigen, die in der ersten Probe (14) enthalten sind, detektiert, detektierten koexistierenden Ionen als einen ersten Auswahlkoeffizienten, K1, für die koexistierenden Ionen der zweiten Ionenauswahlelektrode zu erhalten,

wobei die erste Ionenauswahlelektrode (111; 112; 113) und die Messeinheit (24) jeweils konfiguriert sind, um die zweite Probe (15) zu messen,

wobei die Berechnungseinheit (124, 125, 126) konfiguriert ist, um ein Verhältnis der Konzentration der durch die erste Ionenauswahlelektrode (111; 112; 113) detektierten Zielionen unter den in der zweiten Probe (15) enthaltenen koexistierenden Ionen als einen zweiten Auswahlkoeffizienten, K'1, zu berechnen,

wobei die Berechnungseinheit (124, 125, 126) konfiguriert ist, um ein Ergebnis des Messens der Konzentration der in der ersten Probe (14) enthaltenen koexistierenden Ionen in dem Herstellungsprozess als eine erste koexistierende Ionenkonzentration, C1, zu erhalten,

wobei die Berechnungseinheit (124, 125, 126) konfiguriert ist, um ein Ergebnis der durch die Messeinheit (24) gemessenen Konzentration der in der zweiten Probe (15) enthaltenen koexistierenden Ionen als eine zweite koexistierende Ionenkonzentration, C'1, zu erhalten, und

wobei die Berechnungseinheit (124, 125, 126) konfiguriert ist, um die Konzentration der in der zweiten Probe (15) enthaltenen Zielionen unter Verwendung des ersten Auswahlkoeffizienten, K1, des zweiten Auswahlkoeffizienten, K'1, der ersten koexistierenden Ionenkonzentration, C1, und der zweiten koexistierenden Ionenkonzentration, C'1, zu berechnen.

**6.** Automatische Analysevorrichtung nach Anspruch 5,

wobei die Berechnungseinheit (124, 125, 126) konfiguriert ist, um die erste Ionenauswahlelektrode (111; 112; 113) unter Verwendung eines Ergebnisses der Konzentration der in der ersten Probe (14) enthaltenen Zielionen, die durch die erste Ionenauswahlelektrode (111; 112; 113) detektiert wird, und eines Werts, der vorläufig als die Konzentration der in der ersten Probe (14) enthaltenen Zielionen spezifiziert wird, zu kalibrieren,

wobei die Berechnungseinheit (124, 125, 126) ein Ergebnis der Konzentration der in der zweiten Probe (15) enthaltenen Zielionen, die durch die erste Ionenauswahlelektrode (111; 112; 113) detektiert wird, erhält, und

wobei die Berechnungseinheit (124, 125, 126) konfiguriert ist, um die Konzentration der in der zweiten Probe (15) enthaltenen Zielionen durch Korrigieren eines Ergebnisses der Kalibrierung unter Verwendung des ersten Auswahlkoeffizienten, K1, des zweiten Auswahlkoeffizienten, K'1, der ersten koexistierenden Ionenkonzentration, C1, und der zweiten koexistierenden Ionenkonzentration, C'1, zu berechnen.

7. Automatische Analysevorrichtung nach Anspruch 6,
wobei die Berechnungseinheit (124, 125, 126) konfiguriert ist, um die Konzentration der in der zweiten Probe (15) enthaltenen Zielionen durch Subtrahieren eines Werts, der durch Multiplizieren des ersten Auswahlkoeffizienten, K1, mit der ersten koexistierenden Ionenkonzentration, C1, erhalten wird, von einem Ergebnis der Konzentration der in der zweiten Probe (15) enthaltenen Zielionen, die durch die erste Ionenauswahlelektrode (111; 112; 113) gemessen wird, und durch weiteres Addieren eines Werts, der durch Multiplizieren des zweiten Auswahlkoeffizienten, K'1, mit der zweiten koexistierenden Ionenkonzentration, C'1, erhalten wird, zu dem Ergebnis zu berechnen.

8. Automatische Analysevorrichtung nach Anspruch 1, umfassend eine Schnittstelle, die konfiguriert ist, um ein Detektionsergebnis durch die erste Ionenauswahlelektrode (111; 112; 113) und ein Messergebnis durch die Messeinheit (24) einzugeben,
wobei die Berechnungseinheit (124, 125, 126) konfiguriert ist, um die Konzentration der in der Probe (14; 15) enthaltenen Zielionen unter Verwendung des Detektionsergebnisses durch die erste Ionenauswahlelektrode (111; 112; 113), das durch die Schnittstelle eingegeben wird, und des Messergebnisses durch die Messeinheit (24), das durch die Schnittstelle eingegeben wird, zu berechnen.

9. Automatische Analysevorrichtung nach Anspruch 1 oder 5, umfassend:

eine Speichereinheit (121), die konfiguriert ist, um zeitliche Änderungen der Konzentration der in der Probe (14; 15) enthaltenen koexistierenden Ionen zu speichern; und
eine Ausgabeeinheit, die konfiguriert ist, um zeitliche Änderungen der Konzentration, C1, C'1, der in der Probe (14; 15) enthaltenen koexistierenden Ionen auszugeben.

10. Automatische Analysevorrichtung nach Anspruch 1 oder 5, umfassend:

eine Speichereinheit (121), die konfiguriert ist, um zeitliche Änderungen des Auswahlkoeffizienten der ersten Ionenauswahlelektrode (111; 112; 113) zu speichern; und
eine Ausgabeeinheit, die konfiguriert ist, um zeitliche Änderungen des Auswahlkoeffizienten, K1, K'1, der ersten Ionenauswahlelektrode (111; 112; 113) auszugeben.

11. Automatische Analysevorrichtung nach Anspruch 1 oder 5, umfassend eine Alarmeinheit, die konfiguriert ist, um, wenn die Konzentration, C1, C'1, der in der Probe (14; 15) enthaltenen koexistierenden Ionen oder der Auswahlkoeffizient, K1, K'1, der ersten Ionenauswahlelektrode (111; 112; 113) außerhalb eines vorläufig eingestellten zulässigen Bereichs liegt, einen Alarm zum Melden der Tatsache auszugeben.

12. Automatisches Analyseverfahren zum Messen der Konzentration von in einer Probe enthaltenen Zielionen, wobei das Verfahren Folgendes umfasst:

einen Schritt (S405, S406) des Detektierens der Konzentration der in der Probe (14; 15) enthaltenen Zielionen unter Verwendung einer ersten Ionenauswahlelektrode (111; 112; 113); und
einen Berechnungsschritt (S407) des Berechnens der Konzentration der in der Probe (14; 15) enthaltenen Zielionen,
wobei in dem Berechnungsschritt (S407) ein Detektionsergebnis durch die erste Ionenauswahlelektrode (111; 112; 113) und ein Messergebnis durch eine Messeinheit (24), die die Konzentration, C1, C'1, von in der Probe (14; 15) enthaltenen koexistierenden Ionen misst, erhalten werden,
wobei die Messeinheit (24) die Konzentration, C1, C'1, der in der Probe (14; 15) enthaltenen koexistierenden Ionen unter Verwendung einer Farbreaktion misst,
wobei in dem Berechnungsschritt (S402) ein Verhältnis der durch die erste Ionenauswahlelektrode (111; 112; 113) detektierten koexistierenden Ionen unter denjenigen, die in der Probe (14; 15) enthalten sind, als ein Auswahlkoeffizient, K1, K'1, für die koexistierenden Ionen der ersten Ionenauswahlelektrode (111; 112; 113) berechnet wird, und
wobei in dem Berechnungsschritt (S407) die Konzentration der in der Probe (14; 15) enthaltenen Zielionen unter Verwendung des Detektionsergebnisses durch die erste Ionenauswahlelektrode (111; 112; 113), des Auswahlkoeffizienten, K1, K'1, und der Konzentration, C1, C'1, der durch die Messeinheit (24) gemessenen koexistierenden Ionen berechnet wird.

**13.** Automatisches Analyseverfahren zum Messen der Konzentration von in einer Probe enthaltenen Zielionen nach Anspruch 12,

wobei die erste Ionenauswahlelektrode (111; 112; 113) und die Messeinheit (24) jeweils eine erste Probe (14) und eine zweite Probe (15) als die Probe (14; 15) messen,

wobei in dem Berechnungsschritt (S402) der Auswahlkoeffizient für die in der ersten Probe (14) enthaltenen koexistierenden Ionen als ein erster Probenauswahlkoeffizient, K1, berechnet wird, und der Auswahlkoeffizient für die in der zweiten Probe (15) enthaltenen koexistierenden Ionen als ein zweiter Probenauswahlkoeffizient, K'1, berechnet wird,

wobei die Messeinheit (24) die Konzentration der in der ersten Probe (14) enthaltenen koexistierenden Ionen als eine erste koexistierende Probenionenkonzentration, C1, misst, und die Konzentration der in der zweiten Probe (15) enthaltenen koexistierenden Ionen als eine zweite koexistierende Probenionenkonzentration, C'1, misst,

wobei in dem Berechnungsschritt (S407) die Konzentration der in der zweiten Probe (15) enthaltenen Zielionen unter Verwendung des ersten Probenauswahlkoeffizienten, K1, des zweiten Probenauswahlkoeffizienten, K'1, der ersten koexistierenden Probenionenkonzentration, C1, und der zweiten koexistierenden Probenionenkonzentration, C'1, berechnet wird, und

wobei die Berechnungseinheit (124, 125, 126) die Konzentration der in der zweiten Probe (15) enthaltenen Zielionen durch Addieren eines Werts, der durch Multiplizieren des ersten Probenauswahlkoeffizienten, K1, mit der ersten koexistierenden Probenionenkonzentration, C1, erhalten wird, zu einem Ergebnis der Konzentration der in der zweiten Probe (15) enthaltenen Zielionen, die durch die erste Ionenauswahlelektrode (111; 112; 113) gemessen wird, und durch weiteres Subtrahieren eines Werts, der durch Multiplizieren des zweiten Probenauswahlkoeffizienten, K'1, mit der zweiten koexistierenden Probenionenkonzentration, C'1, erhalten wird, von dem Ergebnis berechnet.

**14.** Automatisches Analyseverfahren nach Anspruch 12,

wobei die Probe (14; 15) eine erste Probe (14) und eine zweite Probe (15) umfasst,

wobei in einem Herstellungsprozess, bevor die erste Probe (14) versandt wird, ein Verhältnis der durch eine zweite Ionenauswahlelektrode, die die Konzentration der Zielionen unter denjenigen, die in der ersten Probe (14) enthalten sind, detektiert, detektierten koexistierenden Ionen als ein erster Auswahlkoeffizient, K1, für die koexistierenden Ionen der zweiten Ionenauswahlelektrode in dem Berechnungsschritt erhalten wird,

wobei die erste Ionenauswahlelektrode (111; 112; 113) und die Messeinheit (24) jeweils die zweite Probe (15) messen,

wobei in dem Berechnungsschritt (S502) ein Verhältnis der Konzentration der durch die erste Ionenauswahlelektrode (111; 112; 113) detektierten Zielionen unter den in der zweiten Probe (15) enthaltenen koexistierenden Ionen als ein zweiter Auswahlkoeffizient, K'1, berechnet wird,

wobei in dem Berechnungsschritt (S507) ein Ergebnis des Messens der Konzentration der in der ersten Probe (14) enthaltenen koexistierenden Ionen in dem Herstellungsprozess als eine erste koexistierende Ionenkonzentration, C1, erhalten wird,

wobei in dem Berechnungsschritt (S507) ein Ergebnis der durch die Messeinheit (24) gemessenen Konzentration der in der zweiten Probe (15) enthaltenen koexistierenden Ionen als eine zweite koexistierende Ionenkonzentration, C'1, erhalten wird, und

wobei in dem Berechnungsschritt (S507) die Konzentration der in der zweiten Probe (15) enthaltenen Zielionen unter Verwendung des ersten Auswahlkoeffizienten, K1, des zweiten Auswahlkoeffizienten, K'1, der ersten koexistierenden Ionenkonzentration, C1, und der zweiten koexistierenden Ionenkonzentration, C'1, berechnet wird.

## Revendications

**1.** Appareil d'analyse automatique qui est configuré pour mesurer la concentration d'ions cibles contenus dans un échantillon, l'appareil comprenant :

une unité de mesure (24) ;

une première électrode de sélection d'ions (111 ; 112 ; 113) qui est configurée pour détecter la concentration des ions cibles contenus dans l'échantillon (14 ; 15) ; et

une unité de calcul (124, 125, 126) qui est configurée pour calculer la concentration des ions cibles contenus dans l'échantillon (14 ; 15),

dans lequel l'unité de calcul (124, 125, 126) est configurée pour obtenir un résultat de détection par la première électrode de sélection d'ions (111 ; 112 ; 113) et un résultat de mesure par l'unité de mesure (24) qui est configurée pour mesurer la concentration, C1, C'1, d'ions coexistants contenus dans l'échantillon (14 ; 15),

dans lequel l'unité de mesure (24) est configurée pour mesurer la concentration, C1, C'1, des ions coexistants contenus dans l'échantillon (14 ; 15) en utilisant une réaction de couleur,

dans lequel l'unité de calcul (124, 125, 126) est configurée pour calculer un rapport des ions coexistants détectés par la première électrode de sélection d'ions (111 ; 112 ; 113) parmi ceux contenus dans l'échantillon (14 ; 15) en tant que coefficient de sélection, K1, K'1, pour les ions coexistants de la première électrode de sélection d'ions (111 ; 112 ; 113), et

dans lequel l'unité de calcul (124, 125, 126) est configurée pour calculer la concentration des ions cibles contenus dans l'échantillon (14 ; 15) en utilisant le résultat de détection par la première électrode de sélection d'ions (111 ; 112 ; 113), le coefficient de sélection, K1, K'1, et la concentration, C1, C'1, des ions coexistants mesurés par l'unité de mesure (24).

2. Appareil d'analyse automatique selon la revendication 1,

dans lequel la première électrode de sélection d'ions (111 ; 112 ; 113) et l'unité de mesure (24) sont configurées pour mesurer chacun d'un premier échantillon (14) et d'un second échantillon (15) en tant qu'échantillon (14 ; 15),

dans lequel l'unité de calcul (124, 125, 126) est configurée pour calculer le coefficient de sélection pour les ions coexistants contenus dans le premier échantillon (14) en tant que premier coefficient de sélection d'échantillon, K1, et est configurée pour calculer le coefficient de sélection pour les ions coexistants contenus dans le second échantillon (15) en tant que second coefficient de sélection d'échantillon, K'1,

dans lequel l'unité de mesure (24) est configurée pour mesurer la concentration des ions coexistants contenus dans le premier échantillon (14) en tant que première concentration d'ions coexistants d'échantillon, C1, et est configurée pour mesurer la concentration des ions coexistants contenus dans le second échantillon (15) en tant que seconde concentration d'ions coexistants d'échantillon, C'1,

dans lequel l'unité de calcul (124, 125, 126) est configurée pour calculer la concentration des ions cibles contenus dans le second échantillon (15) en utilisant le premier coefficient de sélection d'échantillon, K1, le second coefficient de sélection d'échantillon, K'1, la première concentration d'ions coexistants d'échantillon, C1, et la seconde concentration d'ions coexistants d'échantillon, C'1, et

dans lequel l'unité de calcul (124, 125, 126) est configurée pour calculer la concentration des ions cibles contenus dans le second échantillon (15) en ajoutant une valeur obtenue en multipliant le premier coefficient de sélection d'échantillon, K1, par la première concentration d'ions coexistants d'échantillon, C1, à un résultat de la concentration des ions cibles contenus dans le second échantillon (15) mesurée par la première électrode de sélection d'ions (111 ; 112 ; 113) et en soustrayant en outre une valeur obtenue en multipliant le second coefficient de sélection d'échantillon, K'1, par la seconde concentration d'ions coexistants d'échantillon, C'1, du résultat.

3. Appareil d'analyse automatique selon la revendication 2,

dans lequel l'unité de calcul (124, 125, 126) est configurée pour étalonner la première électrode de sélection d'ions (111 ; 112 ; 113) en utilisant un résultat de la concentration des ions cibles contenus dans le premier échantillon (14) détectée par la première électrode de sélection d'ions (111 ; 112 ; 113) et une valeur spécifiée au préalable en tant que concentration des ions cibles contenus dans le premier échantillon (14),

dans lequel l'unité de calcul (124, 125, 126) est configurée pour obtenir un résultat de la concentration des ions cibles contenus dans le second échantillon (15) détectée par la première électrode de sélection d'ions (111 ; 112 ; 113), et

dans lequel l'unité de calcul (124, 125, 126) est configurée pour calculer la concentration des ions cibles contenus dans le second échantillon (15) en corrigeant un résultat de l'étalonnage en utilisant le premier coefficient de sélection d'échantillon, K1, le second coefficient de sélection d'échantillon, K'1, la première concentration d'ions coexistants d'échantillon, C1, et la seconde concentration d'ions coexistants d'échantillon, C'1.

4. Appareil d'analyse automatique selon la revendication 3,

dans lequel la première électrode de sélection d'ions (111 ; 112 ; 113) est configurée pour être échangée entre le moment où l'unité de calcul étalonne la première électrode de sélection d'ions (111 ; 112 ; 113) et le moment où l'unité de calcul obtient un résultat de détection de la concentration, C'1, des ions cibles contenus dans le second échantillon (15), ou

dans lequel la même première électrode de sélection d'ions (111 ; 112 ; 113) est configurée pour être utilisée à chacun du moment où l'unité de calcul étalonne la première électrode de sélection d'ions (111 ; 112 ; 113) et du moment où l'unité de calcul obtient un résultat de détection de la concentration, C'1, des ions cibles contenus dans le second échantillon (15).

5. Appareil d'analyse automatique selon la revendication 1,

dans lequel l'échantillon (14 ; 15) comprend un premier échantillon (14) et un second échantillon (15),
dans lequel dans un processus de fabrication avant que le premier échantillon (14) soit expédié, l'unité de calcul (124, 125, 126) est configurée pour obtenir un rapport des ions coexistants détectés par une seconde électrode de sélection d'ions qui détecte la concentration des ions cibles parmi ceux contenus dans le premier échantillon (14) en tant que premier coefficient de sélection, K1, pour les ions coexistants de la seconde électrode de sélection d'ions,
dans lequel chacune de la première électrode de sélection d'ions (111 ; 112 ; 113) et de l'unité de mesure (24) est configurée pour mesurer le second échantillon (15),
dans lequel l'unité de calcul (124, 125, 126) est configurée pour calculer un rapport de la concentration des ions cibles détectés par la première électrode de sélection d'ions (111 ; 112 ; 113) parmi les ions coexistants contenus dans le second échantillon (15) en tant que second coefficient de sélection, K'1,
dans lequel l'unité de calcul (124, 125, 126) est configurée pour obtenir un résultat de mesure de la concentration des ions coexistants contenus dans le premier échantillon (14) dans le processus de fabrication en tant que première concentration d'ions coexistants, C1,
dans lequel l'unité de calcul (124, 125, 126) est configurée pour obtenir un résultat de la concentration des ions coexistants contenus dans le second échantillon (15) mesurée par l'unité de mesure (24) en tant que seconde concentration d'ions coexistants, C'1, et
dans lequel l'unité de calcul (124, 125, 126) est configurée pour calculer la concentration des ions cibles contenus dans le second échantillon (15) en utilisant le premier coefficient de sélection, K1, le second coefficient de sélection, K'1, la première concentration d'ions coexistants, C1, et la seconde concentration d'ions coexistants, C'1.

6. Appareil d'analyse automatique selon la revendication 5,

dans lequel l'unité de calcul (124, 125, 126) est configurée pour étalonner la première électrode de sélection d'ions (111 ; 112 ; 113) en utilisant un résultat de la concentration des ions cibles contenus dans le premier échantillon (14) détectée par la première électrode de sélection d'ions (111 ; 112 ; 113) et une valeur spécifiée au préalable en tant que concentration des ions cibles contenus dans le premier échantillon (14),
dans lequel l'unité de calcul (124, 125, 126) obtient un résultat de la concentration des ions cibles contenus dans le second échantillon (15) détectée par la première électrode de sélection d'ions (111 ; 112 ; 113), et
dans lequel l'unité de calcul (124, 125, 126) est configurée pour calculer la concentration des ions cibles contenus dans le second échantillon (15) en corrigeant un résultat de l'étalonnage en utilisant le premier coefficient de sélection, K1, le second coefficient de sélection, K'1, la première concentration d'ions coexistants, C1, et la seconde concentration d'ions coexistants, C'1.

7. Appareil d'analyse automatique selon la revendication 6,
dans lequel l'unité de calcul (124, 125, 126) est configurée pour calculer la concentration des ions cibles contenus dans le second échantillon (15) en soustrayant une valeur obtenue en multipliant le premier coefficient de sélection, K1, par la première concentration d'ions coexistants, C1, d'un résultat de la concentration des ions cibles contenus dans le second échantillon (15) mesurée par la première électrode de sélection d'ions (111 ; 112 ; 113) et en ajoutant en outre une valeur obtenue en multipliant le second coefficient de sélection, K'1, par la seconde concentration d'ions coexistants, C'1, au résultat.

8. Appareil d'analyse automatique selon la revendication 1, comprenant une interface qui est configurée pour entrer un résultat de détection par la première électrode de sélection d'ions (111 ; 112 ; 113) et un résultat de mesure par l'unité de mesure (24),
dans lequel l'unité de calcul (124, 125, 126) est configurée pour calculer la concentration des ions cibles contenus dans l'échantillon (14 ; 15) en utilisant le résultat de détection par la première électrode de sélection d'ions (111 ; 112 ; 113) entré par l'intermédiaire de l'interface et le résultat de mesure par l'unité de mesure (24) entré par l'intermédiaire de l'interface.

9. Appareil d'analyse automatique selon la revendication 1 ou 5, comprenant :

une unité de stockage (121) qui est configurée pour stocker des changements temporels de la concentration des ions coexistants contenus dans l'échantillon (14 ; 15) ; et
une unité de sortie qui est configurée pour sortir des changements temporels de la concentration, C1, C'1, des ions coexistants contenus dans l'échantillon (14 ; 15).

10. Appareil d'analyse automatique selon la revendication 1 ou 5, comprenant :

une unité de stockage (121) qui est configurée pour stocker des changements temporels du coefficient de sélection de la première électrode de sélection d'ions (111 ; 112 ; 113) ; et
une unité de sortie qui est configurée pour sortir des changements temporels du coefficient de sélection, K1, K'1, de la première électrode de sélection d'ions (111 ; 112 ; 113).

11. Appareil d'analyse automatique selon la revendication 1 ou 5, comprenant une unité d'alarme qui est configurée pour sortir, lorsque la concentration, C1, C'1, des ions coexistants contenus dans l'échantillon (14 ; 15) ou le coefficient de sélection, K1, K'1, de la première électrode de sélection d'ions (111 ; 112 ; 113) est en dehors d'une plage admissible préétablie, une alarme de notification du fait.

12. Procédé d'analyse automatique pour mesurer la concentration d'ions cibles contenus dans un échantillon, le procédé comprenant :

une étape (S405, S406) de détection de la concentration des ions cibles contenus dans l'échantillon (14 ; 15) en utilisant une première électrode de sélection d'ions (111 ; 112 ; 113) ; et
une étape de calcul (S407) de calcul de la concentration des ions cibles contenus dans l'échantillon (14 ; 15), dans lequel à l'étape de calcul (S407), un résultat de détection par la première électrode de sélection d'ions (111 ; 112 ; 113) et un résultat de mesure par une unité de mesure (24) qui mesure la concentration, C1, C'1, d'ions coexistants contenus dans l'échantillon (14 ; 15) sont obtenus,
dans lequel l'unité de mesure (24) mesure la concentration, C1, C'1, des ions coexistants contenus dans l'échantillon (14 ; 15) en utilisant une réaction de couleur,
dans lequel à l'étape de calcul (S402), un rapport des ions coexistants détectés par la première électrode de sélection d'ions (111 ; 112 ; 113) parmi ceux contenus dans l'échantillon (14 ; 15) est calculé en tant que coefficient de sélection, K1, K'1, pour les ions coexistants de la première électrode de sélection d'ions (111 ; 112 ; 113), et
dans lequel à l'étape de calcul (S407), la concentration des ions cibles contenus dans l'échantillon (14 ; 15) est calculée en utilisant le résultat de détection par la première électrode de sélection d'ions (111 ; 112 ; 113), le coefficient de sélection, K1, K'1, et la concentration, C1, C'1, des ions coexistants mesurés par l'unité de mesure (24).

13. Procédé d'analyse automatique pour mesurer la concentration d'ions cibles contenus dans un échantillon selon la revendication 12,

dans lequel la première électrode de sélection d'ions (111 ; 112 ; 113) et l'unité de mesure (24) mesurent chacun d'un premier échantillon (14) et d'un second échantillon (15) en tant qu'échantillon (14 ; 15),
dans lequel à l'étape de calcul (S402), le coefficient de sélection pour les ions coexistants contenus dans le premier échantillon (14) est calculé en tant que premier coefficient de sélection d'échantillon, K1, et le coefficient de sélection pour les ions coexistants contenus dans le second échantillon (15) est calculé en tant que second coefficient de sélection d'échantillon, K'1,
dans lequel l'unité de mesure (24) mesure la concentration des ions coexistants contenus dans le premier échantillon (14) en tant que première concentration d'ions coexistants d'échantillon, C1, et mesure la concentration des ions coexistants contenus dans le second échantillon (15) en tant que seconde concentration d'ions coexistants d'échantillon, C'1,
dans lequel à l'étape de calcul (S407), la concentration des ions cibles contenus dans le second échantillon (15) est calculée en utilisant le premier coefficient de sélection d'échantillon, K1, le second coefficient de sélection d'échantillon, K'1, la première concentration d'ions coexistants d'échantillon, C1, et la seconde concentration d'ions coexistants d'échantillon, C'1, et
dans lequel l'unité de calcul (124, 125, 126) calcule la concentration des ions cibles contenus dans le second échantillon (15) en ajoutant une valeur obtenue en multipliant le premier coefficient de sélection d'échantillon, K1, par la première concentration d'ions coexistants d'échantillon, C1, à un résultat de la concentration des ions cibles contenus dans le second échantillon (15) mesurée par la première électrode de sélection d'ions (111 ; 112 ; 113) et en soustrayant en outre une valeur obtenue en multipliant le second coefficient de sélection d'échantillon, K'1, par la seconde concentration d'ions coexistants d'échantillon, C'1, du résultat.

14. Procédé d'analyse automatique selon la revendication 12,

dans lequel l'échantillon (14 ; 15) comprend un premier échantillon (14) et un second échantillon (15),
dans lequel dans un processus de fabrication avant que le premier échantillon (14) soit expédié, un rapport des ions coexistants détectés par une seconde électrode de sélection d'ions qui détecte la concentration des ions cibles parmi ceux contenus dans le premier échantillon (14) est obtenu en tant que premier coefficient de sélection, K1, pour les ions coexistants de la seconde électrode de sélection d'ions à l'étape de calcul,
dans lequel chacune de la première électrode de sélection d'ions (111 ; 112 ; 113) et de l'unité de mesure (24) mesure le second échantillon (15),
dans lequel à l'étape de calcul (S502), un rapport de la concentration des ions cibles détectés par la première électrode de sélection d'ions (111 ; 112 ; 113) parmi les ions coexistants contenus dans le second échantillon (15) est calculé en tant que second coefficient de sélection, K'1,
dans lequel à l'étape de calcul (S507), un résultat de mesure de la concentration des ions coexistants contenus dans le premier échantillon (14) dans le processus de fabrication est obtenu en tant que première concentration d'ions coexistants, C1,
dans lequel à l'étape de calcul (S507), un résultat de la concentration des ions coexistants contenus dans le second échantillon (15) mesurée par l'unité de mesure (24) est obtenu en tant que seconde concentration d'ions coexistants, C'1, et
dans lequel à l'étape de calcul (S507), la concentration des ions cibles contenus dans le second échantillon (15) est calculée en utilisant le premier coefficient de sélection, K1, le second coefficient de sélection, K'1, la première concentration d'ions coexistants, C1, et la seconde concentration d'ions coexistants, C'1.

FIG. 1

# FIG. 2

# FIG. 3

123

INPUT UNIT

122

DISPLAY UNIT

124

CPU

MEMORY

CONCENTRATION
OPERATION UNIT

125

CONCENTRATION
OPERATION UNIT

126

# FIG. 4

START

S401 — MEASURE SELECTION COEFFICIENT CALCULATION SAMPLE USING ELECTROLYTE UNIT

S403 — MEASURE CALIBRATION LIQUID AND MEASUREMENT SAMPLE USING MEASUREMENT UNIT

S402 — CALCULATE EACH OF SELECTION COEFFICIENT K1 WHEN CALIBRATING USING CALIBRATION LIQUID AND SELECTION COEFFICIENT K1' WHEN MEASURING MEASUREMENT SAMPLE

S404 — CALCULATE EACH OF COEXISTING ION CONCENTRATION C1 IN CALIBRATION LIQUID AND COEXISTING ION CONCENTRATION C1' IN MEASUREMENT SAMPLE

CALIBRATE CALIBRATION CURVE USING CALIBRATION LIQUID — S405

MEASURE MEASUREMENT SAMPLE — S406

CORRECT MEASUREMENT RESULT — S407

OUTPUT CORRECTION RESULT — S408

END

# FIG. 5

START

S501 — MEASURE SELECTION COEFFICIENT CALCULATION SAMPLE USING ELECTROLYTE UNIT

S503 — MEASURE CALIBRATION LIQUID USING MEASUREMENT UNIT

S502 — CALCULATE EACH OF SELECTION COEFFICIENT K1 AT THE TIME OF VALUE SETTING FOR CALIBRATION LIQUID AND SELECTION COEFFICIENT K1' WHEN MEASURING MEASUREMENT SAMPLE

S504 — CALCULATE EACH OF COEXISTING ION CONCENTRATION C1 AT THE TIME OF VALUE SETTING FOR CALIBRATION LIQUID AND COEXISTING ION CONCENTRATION C1' IN MEASUREMENT SAMPLE

CALIBRATE CALIBRATION CURVE USING CALIBRATION LIQUID — S505

MEASURE MEASUREMENT SAMPLE — S506

CORRECT MEASUREMENT RESULT — S507

OUTPUT CORRECTION RESULT — S508

END

# FIG. 6

START

INPUT K1, C1, K'1 AND C'1 — S601

CALIBRATE CALIBRATION CURVE
USING CALIBRATION LIQUID — S602

MEASURE MEASUREMENT SAMPLE — S603

CORRECT MEASUREMENT RESULT — S604

OUTPUT CORRECTION RESULT — S605

END

# FIG. 7

| Na | K | Cl |

| COEXISTING ION | BASE LIQUID | COEXISTING ION LIQUID | SELECTION COEFFICIENT |
|---|---|---|---|
| A | 141(0) | 145(200) | 0.02 |
| B | 140(0) | 140.5(100) | 0.005 |
| C | ... | ... | ... |
| ... | ... | ... | ... |
| ... | ... | ... | ... |

## FIG. 8

| QC1 | QC2 | CALIBRATION 1 | CALIBRATION 2 | CALIBRATION 3 |
|---|---|---|---|---|

COEXISTING ION CONCENTRATION vs MEASUREMENT DATE

—●— A  —◇— B  —✕— C  - - - LIMIT VALUE

## FIG. 9

| Na | K | Cl |
|---|---|---|

SELECTION COEFFICIENT vs MEASUREMENT DATE

—●— A  —◇— B  —✕— C

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI71995167818 A

- JP H07253409 A